# EUROPEAN PATENT APPLICATION

(11) **EP 1 655 376 A1**
(43) Date of publication of application: **10.05.2006**
(21) Application number: 04734395.9
(22) Date of filing: 21.05.2004
(51) Int. Cl.: C12P 21/00, C12N 15/09

(54) **PROCESS FOR PRODUCING PROTEIN BY CELL-FREE PROTEIN SYNTHESIS SYSTEM AND PROTEN SYNTHESIS REAGENT KIT**

(30) Priority: 22.05.2003 JP 2003145390
(71) Applicant: RIKEN, Wako-shi, Saitama 351-0198 (JP); Taiyo Nippon Sanso Corporation, Tokyo 142-8558 (JP)
(72) Inventor: YOKOYAMA, Shigeyuki, Riken Yokohama Inst. RIKEN, Yokohama-shi, Kanagawa 230-0045 (JP); KIGAWA, Takanori, Riken Yokohama Inst. RIKEN, Yokohama-shi, Kanagawa 230-0045 (JP); NAKAJIMA, Rie, Riken Yokohama Inst., RIKEN, Yokohama-shi, Kanagawa 230-0045 (JP); TANAKA, Akiko, Riken Yokohama Inst., RIKEN, Yokohama-shi, Kanagawa 230-0045 (JP); YOKOYAMA, Jun, Taiyo Nippon Sanso Corporation, Tokyo 1428558 (JP); FUKAI, Yoshihisa, Riken Yokohama Inst., RIKEN, Yokohama-shi, Kanagawa 230-0045 (JP); MATSUMOTO, Takehisa, Riken Yokohama Inst., RIKEN, Yokohama-shi, Kanagawa 230-0045 (JP)
(74) Representative: Woods, Geoffrey Corlett
(86) International application number: PCT/JP2004/007314
(87) International publication number: WO 2004/104210

(57) **Abstract**

This invention provides a process for producing an isotope-labeled protein used as a sample for protein 3D structural analysis via NMR in a cost-effective manner within a short period of time. In this process, a protein is synthesized using, as a substrate, an amino acid mixture that contains a maximum of 19 different types of amino acids selected from the group consisting of alanine, arginine, aspartic acid, cysteine, glutamic acid, glycine, histidine, isoleucine, leucine, lysine, methionine, phenylalanine, proline, serine, threonine, tryptophan, tyrosine, valine, asparagine, and glutamine in a cell-free protein synthesis system.

## Description

### Technical Field

The present invention relates to a process for producing a protein in a cell-free protein synthesis system. In more detail, the present invention relates to a process for producing a protein, particularly a stable isotope-labeled protein, comprising synthesizing a protein using, as a substrate, an amino acid mixture comprising a maximum of 19 different types of amino acids in a cell-free protein synthesis system. The present invention also relates to a reagent kit for protein synthesis for implementing such method.

### Background Art

A cell-free protein synthesis system synthesizes a *protein in vitro* using a cell extract. The cell-free protein synthesis system can use a DNA fragment as an expression template in such a state. This eliminates all the time consuming and laborious steps, such as ligation to a vector, transformation, culturing, harvesting, and lysis of host cells, which had been required in a conventional expression system of a living cell such as *E*. *coli,* yeast, or a cultured cell, thus allowing the ready expression of a protein within a short period of time.

In recent years, so-called functional and structural genomics research, whereby the structures and the functions of genome-encoded proteins are extensively analyzed, has rapidly become advanced, and the number of analyte proteins has increased remarkably. Accordingly, a system for high throughput protein expression that is capable of preparing a large number of protein samples within a short period of time has become necessary. A cell-free protein synthesis system has thus drawn attention.

In the past, 20 different types of protein-constituting amino acids were used as the amino acid substrates necessary for protein synthesis in a cell-free protein synthesis system (for example, JP Patent Publication (Unexamined) Nos. 2000-175695 and 2002-125698). In order to prepare samples for protein 3D structural analysis via NMR, however, use of stable isotope-labeled amino acids was indispensable, and this disadvantageously increased the cost thereof. Among the Algal-derived labeled amino acids used in preparing samples for NMR analysis, cysteine (Cys), tryptophan (Trp), asparagine (Asn), and glutamine (Gln) are disadvantageously eliminated during the process of amino acid hydrolysis, which necessitates supplementation of amino acids. Since these amino acids are more expensive than other amino acids, use of such amino acids is a cause of increased cost.

Examples of conventional techniques for producing a stable isotope-labeled protein include a method wherein an enzyme such as transglutaminase is allowed to act on a protein in the presence of an isotope-labeled ammonium salt, and a functional group of an amino acid residue in the protein is substituted with an isotope-labeled group derived from the isotope-labeled ammonium salt to label the same (JP Patent Publication (Unexamined) No. 2002-332295) and a method wherein an amino acid labeled with a stable isotope such as ¹³C or ¹⁵N is used as a substrate to produce a stable isotope-labeled protein in a cell-free protein synthesis system with lowered enzyme activity except for the protein synthesis system, such as an amino acid biosynthesis system or an amino acid metabolism system (JP Patent No. 3145431). With the former technique, however, an amino acid residue in a protein is labeled with an isotope by allowing an enzyme to act on the protein after protein synthesis. The enzyme catalytic reaction adopted in this technique is different from the reaction adopted in the present invention. Also, amino acid residues located in positions that are in contact with an enzyme are labeled; however, amino acid residues located inside a protein or inside a pocket on the surface cannot be labeled. In the latter technique, 20 different types of amino acids are employed as amino acid substrates, and specific labeling of a given amino acid has not been examined at all.

Accordingly, an object of the present invention is to provide a process for producing a protein, and particularly, a stable isotope-labeled protein as a sample for NMR analysis, in a cost effective manner and a reagent kit for protein synthesis.

### Disclosure of the Invention

The present inventors have conducted concentrated studies in order to attain the above object. They have focused on the fact that asparagine (Asn) and glutamine (Gln) are metabolized and synthesized respectively from aspartic acid (Asp) and glutamic acid (Glu), and they have attempted to synthesize a protein with the use of 18 different types of amino acids excluding asparagine (Asn) and glutamine (Gln) in a cell-free protein synthesis system. As a result, they have found that a protein having activity equivalent to the activity obtained with the use of 20 types of amino acids could be synthesized. They also found that the side chains of asparagine (Asn) and glutamine (Gln) metabolized and synthesized respectively from aspartic acid (Asp) and glutamic acid (Glu) could be specifically labeled without the use of asparagine (Asn) or glutamine (Gln), if an ammonium salt labeled with ¹⁵N is added to the cell-free protein synthesis system. They also found that a main chain of a desired amino acid could be selectively labeled without labeling the side chains of asparagine (Asn) or glutamine (Gln), if a non-labeled ammonium salt is used. The present invention has been completed based on such findings.

Specifically, the present invention includes the following.
(1) A process for producing a protein in a cell-free protein synthesis system, wherein a protein is synthesized using, as an substrate, an amino acid mixture that contains a maximum of 19 different types of amino acids selected from the group consisting of alanine, arginine, aspartic acid, cysteine, glutamic acid, glycine, histidine, isoleucine, leucine, lysine, methionine, phenylalanine, proline, serine, threonine, tryptophan, tyrosine, valine, asparagine, and glutamine.
(2) The process according to (1), wherein the amino acid mixture comprises alanine, arginine, aspartic acid, cysteine, glutamic acid, glycine, histidine, isoleucine, leucine, lysine, methionine, phenylalanine, proline, serine, threonine, tryptophan, tyrosine, and valine.
(3) The process according to (1), wherein the amino acid mixture comprises alanine, arginine, aspartic acid, cysteine, glutamic acid, glycine, histidine, isoleucine, leucine, lysine, methionine, phenylalanine, proline, serine, threonine, tryptophan, tyrosine, valine, and asparagine.
(4) The process according to (1), wherein the amino acid mixture comprises alanine, arginine, aspartic acid, cysteine, glutamic acid, glycine, histidine, isoleucine, leucine, lysine, methionine, phenylalanine, proline, serine, threonine, tryptophan, tyrosine, valine, and glutamine.
(5) The process according to any of (1) to (4), wherein an ammonium salt is added to the cell-free protein synthesis system.
(6) The process according to (5), wherein the ammonium salt is added to the system to a final concentration of 20 mM to 120 mM.
(7) The process according to (5) or (6), wherein the ammonium salt is ammonium acetate.
(8) A process for producing a stable isotope-labeled protein in a cell-free protein synthesis system, wherein an ammonium salt is added to the system, an amino acid mixture comprising a maximum of 19 different types of amino acids selected from the group consisting of alanine, arginine, aspartic acid, cysteine, glutamic acid, glycine, histidine, isoleucine, leucine, lysine, methionine, phenylalanine, proline, serine, threonine, tryptophan, tyrosine, valine, asparagine, and glutamine is used as a substrate, and the ammonium salt and/or at least one amino acid in the mixture are/is labeled with a stable isotope.
(9) The process according to (8), wherein the amino acid mixture comprises alanine, arginine, aspartic acid, cysteine, glutamic acid, glycine, histidine, isoleucine, leucine, lysine, methionine, phenylalanine, proline, serine, threonine, tryptophan, tyrosine, and valine.
(10) The process according to (8), wherein the amino acid mixture comprises alanine, arginine, aspartic acid, cysteine, glutamic acid, glycine, histidine, isoleucine, leucine, lysine, methionine, phenylalanine, proline, serine, threonine, tryptophan, tyrosine, valine, and asparagine.
(11) The process according to (8), wherein the amino acid mixture comprises alanine, arginine, aspartic acid, cysteine, glutamic acid, glycine, histidine, isoleucine, leucine, lysine, methionine, phenylalanine, proline, serine, threonine, tryptophan, tyrosine, valine, and glutamine.
(12) The process according to any of (8) to (11), wherein the ammonium salt is ammonium acetate.
(13) A reagent kit for stable isotope-labeled protein synthesis, which comprises the following components:
   (a) an ammonium salt;
   (b) an amino acid mixture comprising a maximum of 19 different types of amino acids selected from the group consisting of alanine, arginine, aspartic acid, cysteine, glutamic acid, glycine, histidine, isoleucine, leucine, lysine, methionine, phenylalanine, proline, serine, threonine, tryptophan, tyrosine, valine, asparagine, and glutamine; and
   (c) a cell extract for cell-free protein synthesis, and wherein the ammonium salt and/or at least one amino acid in the mixture are/is labeled with a stable isotope.
(14) The kit according to (13), wherein the amino acid mixture comprises alanine, arginine, aspartic acid, cysteine, glutamic acid, glycine, histidine, isoleucine, leucine, lysine, methionine, phenylalanine, proline, serine, threonine, tryptophan, tyrosine, and valine.
(15) The kit according to (13), wherein the amino acid mixture comprises alanine, arginine, aspartic acid, cysteine, glutamic acid, glycine, histidine, isoleucine, leucine, lysine, methionine, phenylalanine, proline, serine, threonine, tryptophan, tyrosine, valine, and asparagine.
(16) The kit according to (13), wherein the amino acid mixture comprises alanine, arginine, aspartic acid, cysteine, glutamic acid, glycine, histidine, isoleucine, leucine, lysine, methionine, phenylalanine, proline, serine, threonine, tryptophan, tyrosine, valine, and glutamine.
(17) The kit according to any of (13) to (16), wherein the ammonium salt is ammonium acetate.

### Brief Description of the Drawings

Fig. 1 shows the results of comparison of the amounts of CAT proteins obtained via cell-free protein synthesis using a mixture of 20 different types of amino acids or a mixture of 18 different types of amino acids as a substrate.
Fig. 2 shows the correlation of the concentration of ammonium acetate added and the amount of protein synthesized.
Fig. 3 shows the ¹⁵N HSQC spectrum of the purified sample of the labeled Ras protein prepared by the process according to the present invention (i.e., dialysis).
Fig. 4 shows the ¹⁵N HSQC spectrum of the ¹⁵N-labeled Ras protein prepared via dialysis with the use of 20 different types of ¹⁵N-labeled amino acids.
Fig. 5 shows the results of comparison of the amounts of GFP proteins obtained via cell-free protein synthesis using a mixture of 20 different types of amino acids or a mixture of 18 different types of amino acids as a substrate in the presence of ammonium acetate at various concentrations.
Fig. 6 shows the results of comparison of the amounts of GFP proteins obtained via cell-free protein synthesis using a mixture of 20 different types of amino acids or a mixture of 18 different types of amino acids (with increased Asp and Glu concentrations) as a substrate in the presence of ammonium acetate at various concentrations.

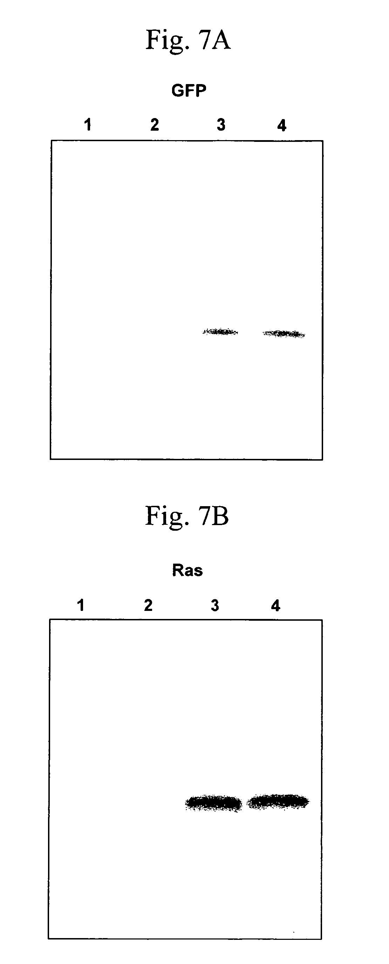

Fig. 7A shows the results of detecting via immunoblotting the GFP proteins synthesized in a cell-free protein synthesis system in the presence of 60 mM ammonium acetate using a mixture of 20 different types of amino acids or a mixture of 18 different types of amino acids (with increased Asp and Glu concentrations) as a substrate (lane 1: without the addition of pQBI T7-GFP (a control for synthesis using a mixture of 20 different types of amino acids); lane 2: without the addition of pQBI T7-GFP (a control for synthesis using a mixture of 18 different types of amino acids); lane 3: synthesis using a mixture of 20 different types of amino acids; and lane 4: synthesis using a mixture of 18 different types of amino acids).
Fig. 7B shows the results of detecting via immunoblotting the Ras proteins synthesized in a cell-free protein synthesis system in the presence of 60 mM ammonium acetate using a mixture of 20 different types of amino acids or a mixture of 18 different types of amino acids (with increased Asp and Glu concentrations) as a substrate (lane 1: without the addition of pK7-NHis-Ras (a control for synthesis using a mixture of 20 different types of amino acids); lane 2: without the addition of pK7-NHis-Ras (a control for synthesis using a mixture of 18 different types of amino acids); lane 3: synthesis using a mixture of 20 different types of amino acids; and lane 4: synthesis using a mixture of 18 different types of amino acids).

Hereafter, the present invention is described in detail. This patent application claims priority from Japanese Patent Application No. 2003-145390 filed on May 22, 2003, and includes part or all of the contents as disclosed in the description and/or drawings thereof.

### 1. Process for producing a protein in a cell-free protein synthesis system

The present invention provides a process for producing a protein in a cell-free protein synthesis system comprising synthesizing a protein using an amino acid mixture comprising a maximum of 19 different types of amino acids as a substrate.

The term "cell-free protein synthesis system" used herein includes a cell-free translation system wherein mRNA information is read and a protein is synthesized on a ribosome and a cell-free transcription/translation system wherein RNA is synthesized from a DNA template.

In the present invention, the term "protein" refers to a polypeptide of any molecular weight constituted by a plurality of amino acid residues. The "protein" particularly refers to a polypeptide having a 3D structure.

The term "amino acid mixture" used herein refers to an amino acid mixture comprising a maximum of 19 different types of amino acids selected from the group consisting of alanine, arginine, aspartic acid, cysteine, glutamic acid, glycine, histidine, isoleucine, leucine, lysine, methionine, phenylalanine, proline, serine, threonine, tryptophan, tyrosine, valine, asparagine, and glutamine. More specifically, the "amino acid mixture" refers to a mixture of 19 different types of amino acids excluding glutamine from the aforementioned list of amino acids, a mixture of 19 different types of amino acids excluding asparagine therefrom, a mixture of 18 different types of amino acids excluding glutamine and asparagine therefrom, and the like. In the present description, all the amino acids are of L-forms. Nonnatural amino acids other than the 20 aforementioned types of protein-constituting amino acids are not included in the definition of "amino acid mixture," although such nonnatural amino acids can be added to the synthesis system of the present invention.

The process of producing a protein in a cell-free protein synthesis system according to the present invention can be carried out in accordance with a known technique with the use of existing materials for cell-free protein synthesis, i.e., a cell extract for cell-free protein synthesis, a template nucleic acid encoding a target protein, or an energy source (a substance containing high-energy phosphate bonds such as ATP, GTP, or creatine phosphate), except that the use of the aforementioned amino acid mixture is alternatively used as an amino acid substrate.

The term "cell extract for cell-free protein synthesis" refers to an extract prepared from plant cells, animal cells, fungal cells, or bacterial cells comprising components that are necessary for a translation system or a transcription/translation system that are involved with protein *in vivo* synthesis, such as a ribosome or tRNA. Specific examples thereof include extracts prepared from *E. coli,* wheat germs, rabbit reticulocytes, mouse L-cells, Ehrlich ascites tumor cells, HeLa cells, CHO cells, and budding yeast. A cell extract can be prepared in accordance with, for example, the method of Pratt, J. M. et al., described in Transcription and translation: A practical approach, 1984, pp. 179-209. Specifically, the aforementioned cells are disrupted using a French press or glass beads, the disrupted cells are homogenized with the addition of a buffer containing several types of salts for solubilizing a protein component or ribosome, and insoluble components are precipitated via centrifugation.

A preferable example of a cell extract is an *E. coli* S30 cell extract. This *E. coli* S30 cell extract can be prepared from the *E*. *coli* A19 strains *(rna* or *met)* in accordance with a conventional technique (Zubay et al., 1973, Ann. Rev. Genet. 7: 267-287). A commercialized cell extract may also be used (available from Promega or Novagen).

The amount of the aforementioned cell extract for cell-free protein synthesis is not particularly limited. For example, such amount is preferably in the range of 10% to 40% by weight based on the total amount of the reaction solution.

The "nucleic acid encoding a target protein" is not particularly limited if such nucleic acid encodes the target protein and comprises an adequate sequence that can be transcribed and/or translated. Such nucleic acid may be any of RNA, mRNA, DNA, or cDNA. Use of DNA necessitates a transcription reaction that requires the use of RNA polymerase or the like, which disadvantageously lowers the yield. When large quantities of proteins are to be synthesized, accordingly, use of mRNA is preferable. DNA or RNA encoding a target protein can be obtained as genomic DNA or mRNA from cells or tissues of eukaryotic or prokaryotic organisms via conventional techniques, such as phenol/chloroform extraction, ethanol precipitation, or cesium chloride density-gradient centrifugation. Alternatively, such DNA or RNA can be synthesized and isolated via cDNA cloning. When the amino acid sequence of the target protein or a nucleotide sequence encoding the same is known, DNA can be chemically synthesized using a DNA synthesizer.

The concentration of the nucleic acid to be added to a reaction solution for cell-free protein synthesis (hereafter it may be referred to as a "reaction solution") can be adequately determined in accordance with, for example, the protein-synthesizing activity of a cell extract for cell-free protein synthesis used or the type of protein to be synthesized. For example, it is approximately 0.1 nM to 10 nM in general.

The energy source in the cell-free protein synthesis system is not particularly limited as long as it can be utilized as an energy source in an organism. Examples of preferable substances include those containing high-energy phosphate bonds such as ATP, GTP, or creatine phosphate. The concentration of the energy source to be added to the reaction solution can be adequately determined in accordance with, for example, the protein-synthesizing activity of a cell extract for cell-free protein synthesis used or the type of protein to be synthesized.

In the present invention, an ammonium salt is preferably added to the cell-free protein synthesis system for the purpose of improving its protein-synthesizing capacity.

Examples of an ammonium salt include ammonium acetate, ammonium benzoate, ammonium citrate, and ammonium chloride, and ammonium acetate is preferable. The concentration of an ammonium salt to be added to the reaction solution is 20 to 120 mM, preferably 20 to 100 mM, and more preferably 40 to 80 mM.

Enzymes involved in ATP regeneration (e.g., a combination of phosphoenolpyruvate with pyruvate kinase or a combination of creatine phosphate with creatine kinase), various types of RNA polymerases (e.g., T7, T3, and SP6 RNA polymerases), or chaperone proteins capable of forming protein 3D structures (e.g., DnaJ, DnaK, GroE, GroEL, GroES, and HSP70) may be optionally added to the aforementioned reaction solution.

The reaction solution can be optionally fortified with nonprotein components. The term "nonprotein components" refers to components that are originally contained in a cell extract for cell-free protein synthesis. Separate addition of such components can improve the protein-synthesizing capacity of the reaction solution. An example thereof is tRNA.

The reaction solution may further comprise various types of additives for protection and/or stabilization of a protein or RNA, if necessary. Examples of such additives include a ribonuclease (RNase) inhibitor (e.g., a placenta RNase inhibitor), a reducing agent (e.g., dithiothreitol), an RNA stabilizer (e.g., spermidine), and a protease inhibitor (e.g., phenylmethanesulfonyl fluoride (PMSF)). The concentration thereof to be added to the reaction solution can be adequately determined in accordance with, for example, the protein-synthesizing activity of a cell extract for cell-free protein synthesis used or the type of protein to be synthesized.

Cell-free protein synthesis may be carried out via a conventional batch or dialysis mode.

When a batch mode is adopted, for example, the reaction solution comprises a nucleic acid encoding a target protein (preferably mRNA), a cell extract for cell-free protein synthesis, the amino acid mixture constituting a target protein, ATP (adenosine 5'-triphosphate), GTP (guanosine 5'-triphosphate), CTP (cytidine 5'-triphosphate), UTP (uridine 5'-triphosphate), a buffer, salts, an RNase inhibitor, and an antibacterial agent. According to need, the reaction solution can further comprise RNA polymerase such as T7 RNA polymerase (when a DNA template is used), tRNA, and the like. In addition, the reaction solution can comprise, for example, a combination of phosphoenolpyruvate with pyruvate kinase or a combination of creatine phosphate with creatine kinase as an ATP regenerating system, polyethylene glycol (e.g., #8000), 3',5'-cAMP, folic acids, a reducing agent (e.g., dithiothreitol), or the like.

Examples of a buffer that can be used include Hepes-KOH and Tris-OAc. Examples of salts that can be used include magnesium acetate, magnesium chloride, potassium acetate, and calcium chloride. Examples of an antibacterial agent that can be used include sodium azide and ampicillin.

The reaction conditions can be adequately determined in accordance with, for example, the cell extract for cell-free protein synthesis used or the type of protein to be synthesized. The reaction temperature is generally 20°C to 40°C, and preferably 23°C to 37°C, and the reaction duration is generally 1 to 5 hours, and preferably 3 to 4 hours.

When a target protein is continuously produced via dialysis, the dialysis internal solution containing the reaction solution used for the aforementioned batch mode is dialyzed against the dialysis external solution existing in an amount of 5 to 10 times larger than the amount of the dialysis internal solution, and the generated target protein is recovered from the dialysis internal solution or the dialysis external solution. A solution prepared by removing a cell extract for cell-free protein synthesis, an RNase inhibitor, a nucleic acid encoding a target protein, and RNA polymerase from the dialysis internal solution can be used as the dialysis external solution. Accordingly, the dialysis external solution may comprise, for example, a buffer, ATP, GTP, CTP, UTP, salts, the amino acid mixture constituting a target protein, a combination of phosphoenolpyruvate with pyruvate kinase as an ATP regenerating system, and an antibacterial agent.

The molecular weight cut-off for the dialysis membrane for separating the dialysis internal solution from the dialysis external solution is 3,500 to 100,000, and preferably 10,000 to 50,000. Dialysis is carried out generally at 20°C to 40°C, and preferably at 23°C to 37°C, while agitating. The dialysis external solution is periodically exchanged with fresh solution (every 24 hours in general). Alternatively, the reaction solution may be periodically (every 24 hours in general) supplemented with another nucleic acid (preferably mRNA). It is preferable to exchange the dialysis external solution with fresh solution when the reaction rate is lowered.

Dialysis can be carried out using a dialyzator that contains the dialysis internal solution separated from the dialysis external solution via a dialysis membrane and that is capable of shaking or agitation (e.g., rotation agitation). Examples of small-scale reaction apparatuses include DispoDialyzer® (Spectrum) and Slidealyzer® (Pierce). An example of a large-scale reaction apparatus is a Spectra/Por® dialysis tubing (Spectrum). The rate of shaking or agitation may be set at a low level. For example, it may be set at 100 to 200 rpm. The reaction duration can be adequately determined while monitoring the generation of the target protein.

The synthesized protein can be relatively easily purified because of the presence of remarkably small amounts and types of contaminants compared with the case of separation of a protein from living cells. Examples of purification techniques include ammonium sulfate or acetone precipitation, acid extraction, anion or cation exchange chromatography, hydrophobic interaction chromatography, affinity chromatography, gel filtration chromatography, hydroxyapatite chromatography, isoelectric chromatography, and chromatofocusing. Purification can be carried out via one of or an adequate combination of these techniques in accordance with the relevant protein characteristics. Alternatively, affinity purification may be adopted, wherein a peptide sequence that is referred to as a "tag" can be previously added to the protein and such tag is specifically recognized and adsorbed. Such purification technique is particularly preferable when obtaining a protein of high purity. Such tag is not particularly limited, and examples of tags that are generally employed include the 6x histidine tag (6xHis), the GST tag, and the maltose-binding tag.

The protein synthesized and purified in the above manner can be identified and quantified via, for example, activity assay, immunological measurement, spectroscopic measurement, or amino-acid analysis, while optionally conducting comparison with a standard sample.

### 2. Process for producing a stable isotope-labeled protein in a cell-free protein synthesis system

The present invention provides a process for producing a stable isotope-labeled protein in a cell-free protein synthesis system, wherein an ammonium salt is added to the system; an amino acid mixture comprising a maximum of 19 different types of amino acids selected from the group consisting of alanine, arginine, aspartic acid, cysteine, glutamic acid, glycine, histidine, isoleucine, leucine, lysine, methionine, phenylalanine, proline, serine, threonine, tryptophan, tyrosine, valine, asparagine, and glutamine is used as a substrate; and the ammonium salt and/or at least one amino acid in the mixture are/is labeled with a stable isotope.

This is a process for synthesizing a stable isotope-labeled protein that is used as a sample for NMR analysis in a cell-free protein synthesis system, and it may be carried out in the same manner as that in 1 above except for the following points. That is, an ammonium salt is added to the system, and the ammonium salt and/or at least one amino acid in the mixture are/is labeled with a stable isotope. The synthesized stable isotope-labeled protein may be purified via the aforementioned techniques, so as to be used as a sample for NMR analysis.

The ammonium salt is not particularly limited as long as it can serve as an ammonia donor in a metabolism system for converting aspartic acid (Asp) and glutamic acid (Glu) into asparagine (Asn) and glutamine (Gln), respectively, represented by the following formulae and can convert the ammonia to side chain amino groups of asparagine and glutamine. Examples thereof include ammonium acetate, ammonium benzoate, ammonium citrate, and ammonium chloride, and ammonium acetate is preferable. The concentration of an ammonium salt to be added to the reaction solution is 20 to 120 mM, preferably 20 to 100 mM, and more preferably 40 to 80 mM. The term "stable isotope" refers to ²H, ¹⁵N, and the like.

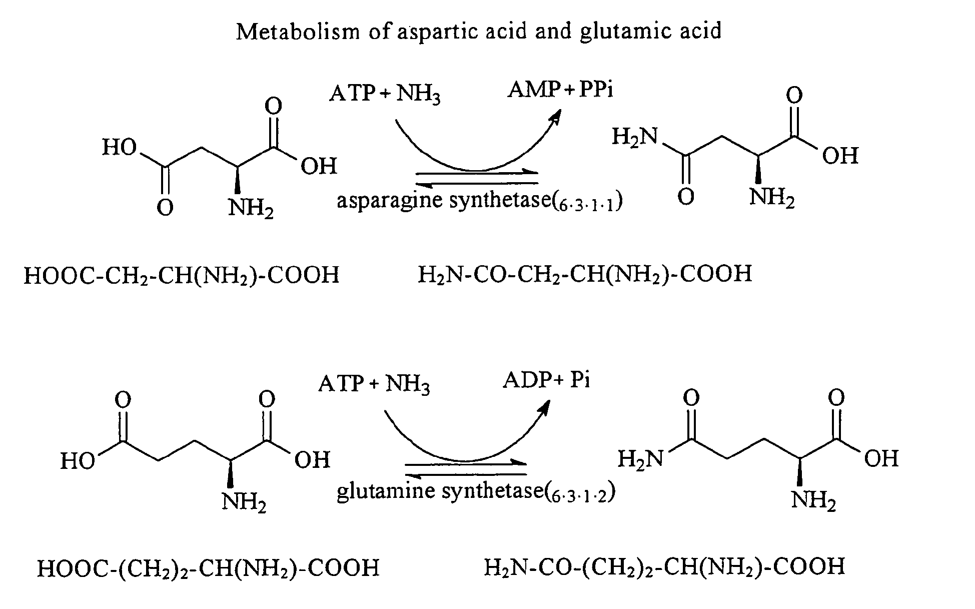

The term "amino acid mixture" refers to an amino acid mixture comprising a maximum of 19 different types of amino acids selected from the group consisting of alanine, arginine, aspartic acid, cysteine, glutamic acid, glycine, histidine, isoleucine, leucine, lysine, methionine, phenylalanine, proline, serine, threonine, tryptophan, tyrosine, valine, asparagine, and glutamine, as described above. More specifically, the "amino acid mixture" refers to a mixture of 19 different types of amino acids excluding glutamine from the aforementioned amino acids, a mixture of 19 different types of amino acids excluding asparagine therefrom, a mixture of 18 different types of amino acids excluding glutamine and asparagine therefrom, and the like.

When a mixture of 18 different types of amino acids is used, for example, such 18 types of amino acids may be mixed with each other. Alternatively, a commercialized Algal mixture may be used, and amino acids such as cysteine or tryptophan that are not included therein may be adequately added to bring the total number of amino acid types to 18. The term "stable isotope" refers to ²H, ¹³C, ¹⁵N, and the like.

Analysis of protein structure often requires the use of many types of labeled proteins. In such a case, several types of labeled amino acids are preferably combined to prepare an amino acid mixture as a substrate. Accordingly, it is sufficient if at least one type of amino acid in the amino acid mixture is labeled. All types of amino acids may also be labeled. The number and the types of amino acids to be labeled may be adequately determined in accordance with, for example, the type of the analyte protein or the purpose of analysis.

### 3. Reagent kit for stable isotope-labeled protein synthesis

As described above, the ammonium salt labeled with a stable isotope can specifically label the side chains of asparagine (Asn) and glutamine (Gln) metabolized and synthesized respectively from aspartic acid (Asp) and glutamic acid (Glu) upon protein synthesis in a cell-free protein synthesis system where an amino acid mixture that does not contain asparagine (Asn) or glutamine (Gln) is used as a substrate. In contrast, use of a non-labeled ammonium salt enables the preparation of a sample wherein labeling of the asparagine (Asn) and glutamine (Gln) side chains is inhibited and the main chain of the desired amino acid is selectively labeled. Accordingly, a stable isotope-labeled or non-labeled ammonium salt, preferably stable isotope-labeled or non-labeled ammonium acetate, may be adequately combined with a stable isotope-labeled amino acid mixture according to need, and the resultant can be provided in the form of a kit for stable isotope-labeled protein synthesis together with other components necessary for protein production in a cell-free protein synthesis system.

For example, the kit for stable isotope-labeled protein synthesis comprises the following components:
(a) an ammonium salt;
(b) an amino acid mixture comprising a maximum of 19 different types of amino acids selected from the group consisting of alanine, arginine, aspartic acid, cysteine, glutamic acid, glycine, histidine, isoleucine, leucine, lysine, methionine, phenylalanine, proline, serine, threonine, tryptophan, tyrosine, valine, asparagine, and glutamine; and
(c) a cell extract for cell-free protein synthesis, and wherein the ammonium salt and/or at least one amino acid in the mixture are/is labeled with a stable isotope.

In the kit of the present invention, at least one type of amino acid in the amino acid mixture may be labeled with a stable isotope.

The type of ammonium salt and the definition of an amino acid mixture are as described in 2 above.

The kit of the present invention can comprise ribonucleotides, such as ATP, GTP, CTP, or UTP, and a buffer for adjusting the pH of the substrate solution.

### Preferred Embodiments of the Invention

The present invention is hereafter described in greater detail with reference to the following examples, although the technical scope of the present invention is not limited thereto.

### Example 1: Cell-free protein synthesis using 18 different types of amino acids (batch mode)

The reaction solution having the following composition (total amount: 30 µl) was incubated at 37°C for 1 hour to carry out protein synthesis. "pK7-CAT" in the following reaction solution refers to a CAT expression vector prepared in accordance with the method described in Kim et al., 1996, Eur. J. Biochem. 239: 881-886.

**(Composition of reaction solution for cell-free protein synthesis by the batch mode)**

| | |
|---|---|
| HEPES-KOH (pH 7.5) | 60 mM |
| Dithiothreitol | 1.8 mM |
| ATP | 1.3 mM |
| CTP, GTP, UTP | 0.9 mM each |
| Creatine phosphate | 80 mM |
| Creatine kinase (Roche) | 250 µg/ml |
| Polyethylene glycol 8000 | 4.0% |
| 3',S'-cyclic AMP | 0.66 mM |
| L(-)-5-formyl-5,6,7,8-tetrahydrofolic acid | 36 µM |
| Total tRNA from *E. coli* (MRE600, Roche) | 175 5 µg/ml |
| Ammonium acetate | 80 mM |
| Magnesium acetate | 10.7 mM |
| 20 types of or 18 types (excluding Asn and Gln) of amino acids | 1 mM |
| T7 RNA polymerase | 66.7 µg/ml |
| *E. coli* S30 extract (Roche) | 7.2 µl |
| 0.2 µg/ml Template DNA (pK7-CAT) | |

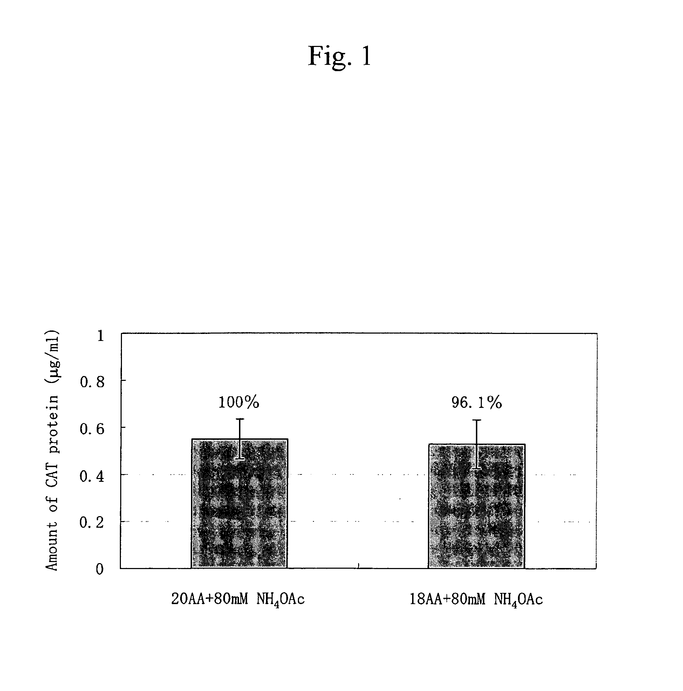

After the reaction, the reaction solution was placed on ice to terminate the reaction, and the chloramphenicol acetyl transferase (CAT) protein contained in the reaction solution was quantified in the following manner in accordance with Shaw, 1975, Methods Enzymol, pp. 735-755. Specifically, CAT-mediated acetylation of chloramphenicol was carried out using acetyl coenzyme A and chloramphenicol substrates, and the resulting reduced coenzyme A was quantified based on color development with the use of 5,5'-dithiobis-2-nitrobenzoic acid (DTNB). Based on an increase in the absorbance at 37°C and 412 nm per unit time, CAT activity was quantified, and the amount of the CAT protein was determined based on the quantified value as an indicator. The amounts of protein synthesized from 20 different types of amino acids and from 18 different types of amino acids were 0.551 mg and 0.529 mg, respectively. There was no significant difference therebetween (Fig. 1). This indicates that protein synthesis is feasible in a cell-free protein synthesis system where a mixture of 18 different types of amino acids excluding asparagine (Asn) and glutamine (Gln) is used.

### Example 2: Examination of ammonium acetate concentration (batch mode)

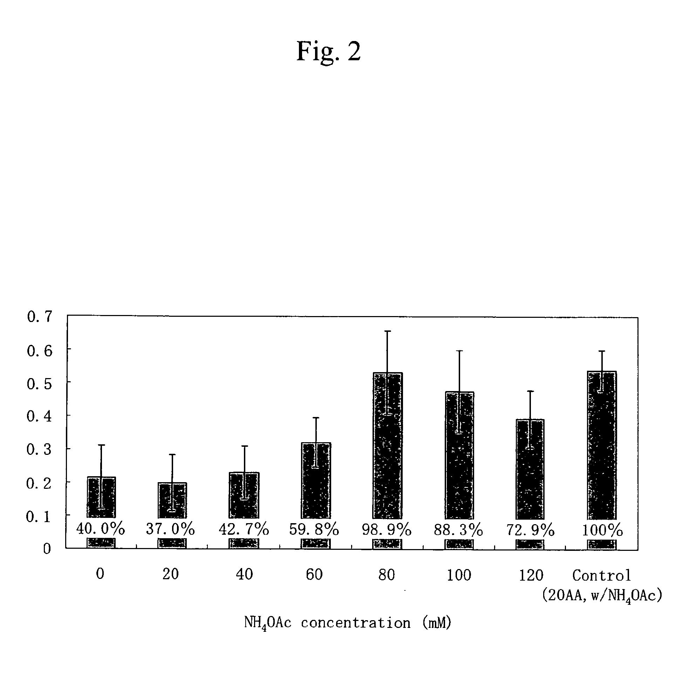

Protein synthesis was carried out in the same manner as in Example 1 except for the use of ammonium sulfate at various concentrations in the reaction solution for cell-free protein synthesis in the aforementioned batch mode, and the amount of CAT protein was quantified. The results are shown in Fig. 2. As the concentration of ammonium acetate increased, the amount of CAT protein increased, and the amount of CAT protein reached the maximal level at 80 mM ammonium acetate.

### Example 3: Cell-free protein synthesis using 18 different types of amino acids (dialysis) (1) Synthesis of Ras protein

"pK7-NHis-Ras" (0.012 ml), which is a 1 mg/ml circular double-stranded DNA expression vector, comprising a Ras protein-encoding gene as template DNA and consisting of the nucleotide sequence as shown in SEQ ID NO: 1 was added to the dialysis internal solution shown in (i) below to bring the total amount thereof to 3 ml. This dialysis internal solution was placed in the DispoDialyzer CE (molecular weight limits: 10,000 and 50,000, Spectrum), suspended in 30 ml of the dialysis external solution shown in (ii) below, and shook at 30°C for 4 hours in shaking culture system for test tube. Thus, protein synthesis was carried out.

### (Composition of reaction solution for cell-free protein synthesis via dialysis)

### (i) Dialysis internal solution:

| | |
|---|---|
| HEPES-KOH (pH 7.5) | 60 mM |
| Dithiothreitol | 1.8 mM |
| ATP | 1.3 mM |
| CTP, GTP, UTP | 0.9 mM each |
| Creatine phosphate | 80 mM |
| Creatine kinase (Roche) | 250 µg/ml |
| Polyethylene glycol 8000 | 4.0% |
| 3',5'-cyclic AMP | 0.66 mM |
| L(-)-5-formyl-5,6,7,8-tetrahydrofolic acid | 36µM |
| Total tRNA from *E*. *coli* (MRE600, Roche) | 175 /µg/ml |
| ¹⁵N-labeled ammonium acetate | 80 mM |
| Magnesium acetate | 10.7 mM |
| 20 types of or 18 types (excluding Asn and Gln) of amino acids | 2 mM |
| Sodium azide | 0.05% |
| T7 RNA polymerase | 66.7 µg/ml |
| *E. coli* S30 extract (Roche) | 0.9 ml |
| 1 mg/ml Template DNA (pK7-NHis-Ras) | |

### (ii) Dialysis external solution:

| | |
|---|---|
| HEPES-KOH (pH 7.5) | 60 mM |
| Dithiothreitol | 1.8 mM |
| ATP | 1.3 mM |
| CTP, GTP, UTP | 0.9 mM each |
| Creatine phosphate | 80 mM |
| Polyethylene glycol 8000 | 4.0% |
| 3',5'-cyclic AMP | 0.66 mM |
| L(-)-5-formyl-5,6,7,8-tetrahydrofolic acid | 36 µM |
| ¹⁵N-labeled ammonium acetate | 80 mM |
| Magnesium acetate | 10.7 mM |
| 20 types of or 18 (excluding Asn and Gln) types of amino acids | 2 mM |
| Sodium azide | 0.05% |

### (2) Purification of ¹⁵N-labeled Ras protein

The ¹⁵N-labeled Ras protein synthesized in the above manner was purified. In protein purification, the affinity of the histidine tags for nickel was utilized, and the purification procedure was carried out at 4°C. After the completion of synthesis, 3 ml of the reaction solution was first diluted with 4.2 ml of washing buffer (50 mM sodium phosphate (pH 8.0)/300 mM sodium chloride/10 mM imidazole) and recovered. Centrifugation was then carried out at 1,960xg for 5 minutes to remove the precipitate. Subsequently, the obtained supernatant was allowed to pass through a 0.8 ml-column of Ni-NTA resin (Qiagen) and adsorb thereon, and contaminants were then removed by causing 9.6 ml of washing buffer to flow therein. Finally, 4 ml of elution buffer (50 mM sodium phosphate (pH 8.0)/300 mM sodium chloride/500 mM imidazole) was allowed to pass though the column to release the sample from the resin. Thus, 0.88 mg of purified sample was obtained.

### (3) Preparation of sample for NMR analysis

In order to convert the purified sample to a solvent suitable for NMR analysis, the sample was converted to a solution comprising 20 mM sodium phosphate (pH 6.5), 100 mM sodium chloride, 5 mM magnesium chloride, 5 mM DTT, and 0.01% by weight NaN₃. Thereafter, the sample was concentrated to 0.25 ml (sample concentration: 0.28 mM). An ultrafiltration apparatus (VivaSpin 2, Sartorius) was used for the above operations. Finally, 0.03 ml of deuterium oxide was added to prepare a sample for NMR analysis.

### (4) NMR analysis

A Shigemi symmetrical microtube (for a 5 mm probe) was used as a sampling tube for NMR analysis. NMR analysis was carried out in a 700 MHz NMR apparatus (Advance 700, Bruker) at 25°C. Evaluation was carried out based on the ¹H-¹⁵N two-dimensional HSQC spectra (hereafter abbreviated as "¹⁵N HSQC spectra"). The conditions thereof are shown in Table 1.

**Table 1 (Conditions for NMR analysis)**

| Spectrum | Number of scans | Center frequencies | Spectral range | Data points |
|---|---|---|---|---|
| ¹H-¹⁵N HSQC | | ¹H: 700.2332911MHz | ¹H:9765.625 Hz | ¹H: 1024 |
| | | ¹⁵N: 70.9620093MHz | ¹⁵N:2593.377617 Hz | ¹⁵N: 128 |

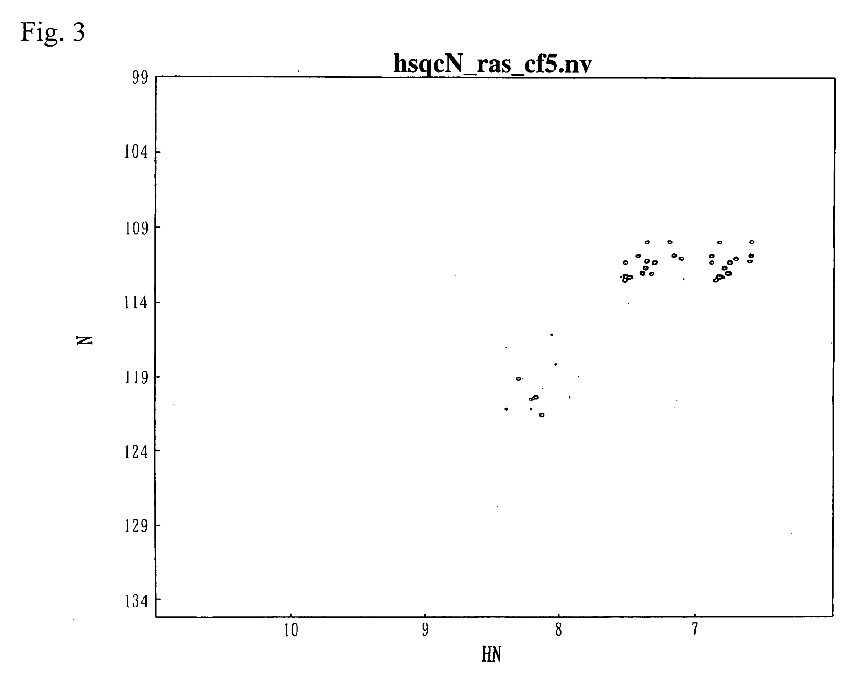

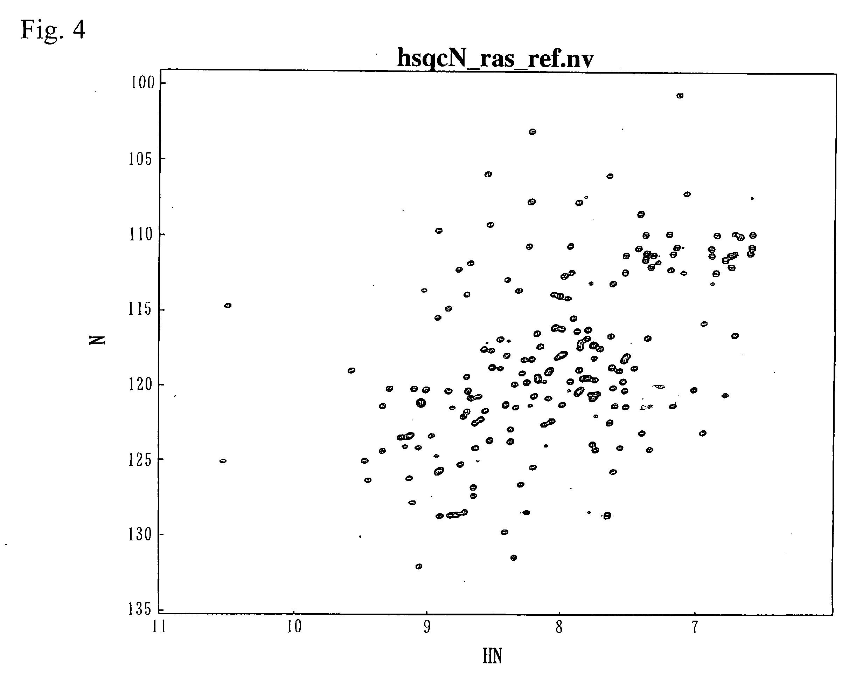

As a result of NMR analysis, the ¹⁵N HSQC spectrum showed amino proton signals scattered in a range from 6.5 ppm to 7.5 ppm as shown in Fig. 3. Such signal appearance is more characteristic for a peak derived from the side chain amino groups of asparagine (Asn) and glutamine (Gln), than the ¹⁵N HSQC spectrum (Fig. 4) of the homogenous ¹⁵N-labeled Ras protein prepared using 20 different types of ¹⁵N-labeled amino acids via dialysis. In the obtained Ras protein, accordingly, it was found that the side chains of asparagine (Asn) and glutamine (Gln) modified by stable isotope-labeled ammonium acetate, which had been added to the synthesis system, were selectively labeled.

### Example 4: Examination of ammonium acetate concentration (batch mode)

The reaction solution having the following composition (total amount: 30 µl) was used to carry out protein synthesis. "pQBI T7-GFP" in the reaction solution refers to a GFP expression vector, which was purchased from Wako Pure Chemical Industries, Ltd.

### (Composition of reaction solution for cell-free protein synthesis by the batch mode)

| | |
|---|---|
| HEPES-KOH (pH 7.5) | 60 mM |
| Dithiothreitol | 1.8 mM |
| ATP | 1.3 mM |
| CTP, GTP, UTP | 0.9 mM each |
| Creatine phosphate | 80 mM |
| Creatine kinase (Roche) | 250 µg/ml |
| Polyethylene glycol 8000 | 4.0 % |
| 3',5'-cyclic AMP | 0.66 mM |
| L(-)-5-formyl-5,6,7,8-tetrahydrofolic acid | 36 µM |
| Total tRNA from *E. coli* (MRE600, Roche) | 175 µg/ml |
| Ammonium acetate | 0 to 120 mM |
| Magnesium acetate | 10.7 mM |
| 20 types of or 18 (excluding Asn and Gln) types of amino acids | 1 mM |
| T7 RNA polymerase | 66.7 µg/ml |
| *E. coli* S30 extract (Roche) | 7.2 µl |
| 2 µg/ml Template DNA (pQBI T7-GFP) | |

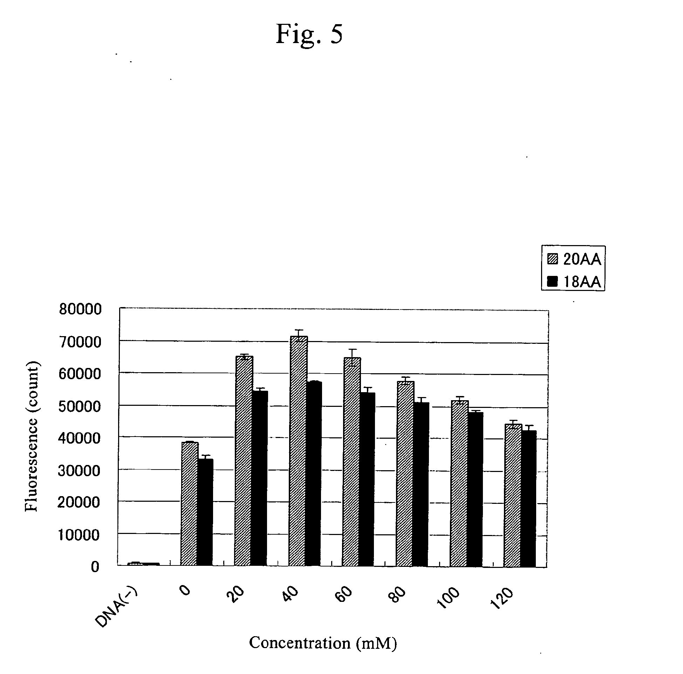

The reaction solution was allowed to stand on a heat block at 37°C for 1 hour. The reaction solution was further allowed to stand at 4°C for an additional 24 hours and then diluted 20-fold with PBS. The fluorescence intensity of GFP was measured at an excitation wavelength of 485 nm and at a detection wavelength of 510 nm. Data are shown in terms of the amount of fluorescence per reaction solution (Fig. 5).

The maximal amount of GFP synthesized was attained at an ammonium acetate concentration of 40 mM with the use of 20 or 18 different types of amino acids.

### Example 5: Examination of Asp, Glu, and ammonium acetate concentrations in cell-free protein synthesis using 18 different types of amino acids (batch mode)

The reaction solution having the following composition (total amount: 30 µl) was used to carry out protein synthesis. Among the 18 types of amino acids used for synthesis, the concentrations of aspartic acid (Asp) and glutamic acid (Glu) were set at 1.5 times higher than the usual levels (i.e., 1.5 mM).

"pQBI T7-GFP" in the following reaction solution refers to a GFP expression vector, which was purchased from Wako Pure Chemical Industries, Ltd.

### (Composition of reaction solution for cell-free protein synthesis by the batch mode)

| | |
|---|---|
| HEPES-KOH (pH 7.5) | 60 mM |
| Dithiothreitol | 1.8 mM |
| ATP | 1.3 mM |
| CTP, GTP, UTP | 0.9 mM each |
| Creatine phosphate | 80 mM |
| Creatine kinase (Roche) | 250 µg/ml |
| Polyethylene glycol 8000 | 4.0% |
| 3',5'-cyclic AMP | 0.66 mM |
| L(-)-5-formyl-5,6,7,8-tetrahydrofolic acid | 36 µM |
| Total tRNA from *E*. *coli* (MRE600, Roche) | 175 µg/ml |
| Ammonium acetate | 0 to 120 mM |
| Magnesium acetate | 10.7 mM |
| 20 or 18 different types of amino acids excluding Asn and Gln | 1 mM each |
| Asp, Glu | 1.5 mM each |
| 16 types of amino acids excluding Asp and Glu | 1 mM each |
| T7 RNA polymerase | 66.7 µg/ml |
| *E. coli* S30 extract (Roche) | 7.2 µl |
| 2 µg/ml Template DNA (pQBI T7-GFP) | |

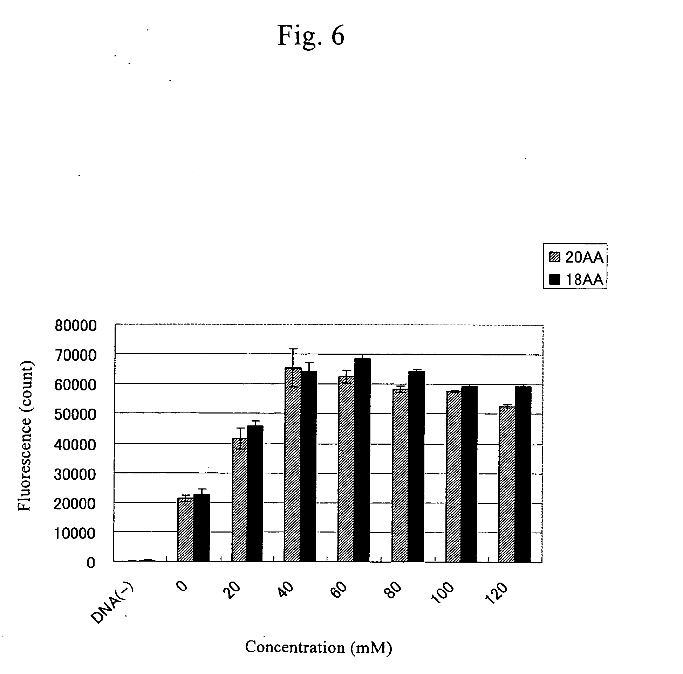

The reaction solution was allowed to stand on a heat block at 37°C for 1 hour. The reaction solution was further allowed to stand at 4°C for an additional 24 hours and then diluted 20-fold with PBS. The fluorescence intensity of GFP was measured at an excitation wavelength of 485 nm and at a detection wavelength of 510 nm. Data are shown in terms of the amount of fluorescence per reaction solution (Fig. 6).

When 20 different types of amino acids were used, the maximal amount of GFP synthesized was attained at an ammonium acetate concentration of 40 mM, and when 18 different types of amino acids (the concentration of aspartic acid (Asp) and that of glutamic acid (Glu) were set at 1.5 times higher than the usual levels (i.e., 1.5 mM)) were used, the maximal amount of GFP synthesized was attained at an ammonium acetate concentration of 60 mM. When 18 different types of amino acids were used, the amount of GFP synthesized at peak was equivalent to that attained with the use of 20 different types of amino acids. This indicates that addition of aspartic acid (Asp) and glutamic acid (Glu) in amounts 1.5 times higher than the usual levels results in the synthesis of amino acid in an amount equivalent to that attained via a conventional technique.

### Example 6: Comparison of amounts of proteins synthesized in cell-free protein synthesis using 20 or 18 different types of amino acids (batch mode)

Amino acids (20 different types, concentration of each amino acid: 1.0 mM) or 18 different types of amino acids (concentration of each amino acid: 1.0 mM; concentrations of aspartic acid (Asp) and glutamic acid (Glu): 1.5 mM) were used, and GFP proteins and Ras proteins were synthesized with 60 mM ammonium acetate concentration.

The composition of the reaction solution is shown below. "pQBI T7-GFP" and "pK7-NHis-Ras" in the reaction solution are as defined above.

### (Composition of reaction solution for cell-free protein synthesis by the batch mode)

| | |
|---|---|
| HEPES-KOH (pH 7.5) | 60 mM |
| Dithiothreitol | 1.8 mM |
| ATP | 1.3 mM |
| CTP, GTP, UTP | 0.9 mM each |
| Creatine phosphate | 80 mM |
| Creatine kinase (Roche) | 250 µg/ml |
| Polyethylene glycol 8000 | 4.0 % |
| 3',5'-cyclic AMP | 0.66 mM |
| L(-)-5-formyl-5,6,7,8-tetrahydrofolic acid | 36 µM |
| Total tRNA from *E. coli* (MRE600, Roche) | 175 µg/ml |
| Ammonium acetate | 60 mM |
| Magnesium acetate | 10.7 mM |
| 20 or 18 different types of amino acids excluding Asn and Gln | 1 mM each |
| Asp, Glu | 1.5mM each |
| 16 different types of amino acids excluding Asp and Glu | 1 mM each |
| T7 RNA polymerase | 66.7 µg/ml |
| *E. coli* S30 extract (Roche) | 7.2 µl |
| 2 µg/ml Template DNA (pQBI T7-GFP or pK7-NHis-Ras) | |

The reaction solution was allowed to stand on a heat block at 37°C for 1hour. After separation via SDS-PAGE, protein detection was carried out via immunoblotting with the use of the anti-GFP antibody (GFP) or the anti-HAT antibody (Ras) (Fig. 7). The amounts of both proteins synthesized with the use of 18 different types of amino acids were equivalent to that attained with the use of 20 different types of amino acids.

All publications, patents, and patent applications cited herein are incorporated herein by reference in their entirety.

### Industrial Applicability

When protein synthesis was carried out with the use of a mixture of 18 different types of amino acids excluding asparagine (Asn) and glutamine (Gln) as a substrate in a cell-free protein synthesis system, a protein having activity equivalent to that attained with the use of 20 different types of amino acids was synthesized, as described above. When the ¹⁵N-labeled ammonium salt or non-labeled ammonium salt was added to the cell-free protein synthesis system, labeling of asparagine (Asn) and glutamine (Gln) side chains was controlled. Therefore, the present invention enables production of a stable isotope-labeled protein in a cost-effective manner and thus is very useful for high-throughput analysis of protein 3D structure.

## Claims

1. A process for producing a protein in a cell-free protein synthesis system, wherein a protein is synthesized using, as an substrate, an amino acid mixture that contains a maximum of 19 different types of amino acids selected from the group consisting of alanine, arginine, aspartic acid, cysteine, glutamic acid, glycine, histidine, isoleucine, leucine, lysine, methionine, phenylalanine, proline, serine, threonine, tryptophan, tyrosine, valine, asparagine, and glutamine.

2. The process according to claim 1, wherein the amino acid mixture comprises alanine, arginine, aspartic acid, cysteine, glutamic acid, glycine, histidine, isoleucine, leucine, lysine, methionine, phenylalanine, proline, serine, threonine, tryptophan, tyrosine, and valine.

3. The process according to claim 1, wherein the amino acid mixture comprises alanine, arginine, aspartic acid, cysteine, glutamic acid, glycine, histidine, isoleucine, leucine, lysine, methionine, phenylalanine, proline, serine, threonine, tryptophan, tyrosine, valine, and asparagine.

4. The process according to claim 1, wherein the amino acid mixture comprises alanine, arginine, aspartic acid, cysteine, glutamic acid, glycine, histidine, isoleucine, leucine, lysine, methionine, phenylalanine, proline, serine, threonine, tryptophan, tyrosine, valine, and glutamine.

5. The process according to any of claims 1 to 4, wherein an ammonium salt is added to the cell-free protein synthesis system.

6. The process according to claim 5, wherein the ammonium salt is added to the system to a final concentration of 20 mM to 120 mM.

7. The process according to claim 5 or 6, wherein the ammonium salt is ammonium acetate.

8. A process for producing a stable isotope-labeled protein in a cell-free protein synthesis system, wherein an ammonium salt is added to the system, an amino acid mixture comprising a maximum of 19 different types of amino acids selected from the group consisting of alanine, arginine, aspartic acid, cysteine, glutamic acid, glycine, histidine, isoleucine, leucine, lysine, methionine, phenylalanine, proline, serine, threonine, tryptophan, tyrosine, valine, asparagine, and glutamine is used as a substrate, and the ammonium salt and/or at least one amino acid in the mixture are/is labeled with a stable isotope.

9. The process according to claim 8, wherein the amino acid mixture comprises alanine, arginine, aspartic acid, cysteine, glutamic acid, glycine, histidine, isoleucine, leucine, lysine, methionine, phenylalanine, proline, serine, threonine, tryptophan, tyrosine, and valine.

10. The process according to claim 8, wherein the amino acid mixture comprises alanine, arginine, aspartic acid, cysteine, glutamic acid, glycine, histidine, isoleucine, leucine, lysine, methionine, phenylalanine, proline, serine, threonine, tryptophan, tyrosine, valine, and asparagine.

11. The process according to claim 8, wherein the amino acid mixture comprises alanine, arginine, aspartic acid, cysteine, glutamic acid, glycine, histidine, isoleucine, leucine, lysine, methionine, phenylalanine, proline, serine, threonine, tryptophan, tyrosine, valine, and glutamine.

12. The process according to any of claims 8 to 11, wherein the ammonium salt is ammonium acetate.

13. A reagent kit for stable isotope-labeled protein synthesis, which comprises the following components:
(a) an ammonium salt;
(b) an amino acid mixture comprising a maximum of 19 different types of amino acids selected from the group consisting of alanine, arginine, aspartic acid, cysteine, glutamic acid, glycine, histidine, isoleucine, leucine, lysine, methionine, phenylalanine, proline, serine, threonine, tryptophan, tyrosine, valine, asparagine, and glutamine; and
(c) a cell extract for cell-free protein synthesis, and wherein the ammonium salt and/or at least one amino acid in the mixture are/is labeled with a stable isotope.

14. The kit according to claim 13, wherein the amino acid mixture comprises alanine, arginine, aspartic acid, cysteine, glutamic acid, glycine, histidine, isoleucine, leucine, lysine, methionine, phenylalanine, proline, serine, threonine, tryptophan, tyrosine, and valine.

15. The kit according to claim 13, wherein the amino acid mixture comprises alanine, arginine, aspartic acid, cysteine, glutamic acid, glycine, histidine, isoleucine, leucine, lysine, methionine, phenylalanine, proline, serine, threonine, tryptophan, tyrosine, valine, and asparagine.

16. The kit according to claim 13, wherein the amino acid mixture comprises alanine, arginine, aspartic acid, cysteine, glutamic acid, glycine, histidine, isoleucine, leucine, lysine, methionine, phenylalanine, proline, serine, threonine, tryptophan, tyrosine, valine, and glutamine.

17. The kit according to any of claims 13 to 16, wherein the ammonium salt is ammonium acetate.


SEQUENCE LISTING

<110> RIKEN

<110> NIPPON SANSO CORPORATION

<120> PROCESS FOR PRODUCING PROTEIN IN CELL-FREE PROTEIN SYNTHESIS SYSTEM AND REAGENT

KIT FOR PROTEIN SYNTHESIS

<130> PH-2164-PCT

<150> JP 2003-145390

<151> 2003-05-22

<160> 1

<170> PatentIn Ver. 2.1

<210> 1

<211> 3396

<212> DNA

<213> Artificial Sequence

<220>

<223> Description of Artificial Sequence: synthetic DNA

<400> 1

tcgacggatc gttccactga gcgtcagacc ccgtagaaaa gatcaaagga tcttcttgag 60

atcctttttt tctgcgcgta atctgctgct tgcaaacaaa aaaaccaccg ctaccagcgg 120

tggtttgttt gccggatcaa gagctaccaa ctctttttcc gaaggtaact ggcttcagca 180

gagcgcagat accaaatact gtccttctag tgtagccgta gttaggccac cacttcaaga 240
actctgtagc accgcctaca tacctcgctc tgctaatcct gttaccagtg gctgctgcca 300
gtggcgataa gtcgtgtctt accgggttgg actcaagacg atagttaccg gataaggcgc 360
agcggtcggg ctgaacgggg ggttcgtgca cacagcccag cttggagcga acgacctaca 420
ccgaactgag atacctacag cgtgagctat gagaaagcgc cacgcttccc gaagggagaa 480
aggcggacag gtatccggta agcggcaggg tcggaacagg agagcgcacg agggagcttc 540
cagggggaaa cgcctggtat ctttatagtc ctgtcgggtt tcgccacctc tgacttgagc 600
gtcgattttt gtgatgctcg tcaggggggc ggagcctatg gaaaaacgcc agcaacgcgg 660
cctttttacg gttcctggcc ttttgctggc cttttgctca catgttcttt cctgcgttat 720
cccctgattc tgtggataac cgtattaccg cctttgagtg agctgatacc gctcgccgca 780
gccgaacgac cgagcgcagc gagtcagtga gcgaggaagc ggaagagcgc cgcacattca 840
gcagcgtttt tcagcgcgtt ttcgatcagc gtttcaatgt tggtatcaac accaggttta 900
actttgaact tatcggcact gacggttact gattttgaac ttttgctttg ccacggaacg 960
gtctgcgttg tcgggaagat gcgtgatctg atccttcaac tcagcaaaag ttcgccaata 1020
cgcaaaccgc ctctccccgc gcgttggccg attcattaat gcagctggca cgacaggttt 1080
cccgactgga attcagatct cgatcccgcg aaattaatac gactcactat agggagacca 1140
caacggtttc cctctagaaa taattttgtt taactttaag aaggagatat acatatgaaa 1200
gatcatctca tccacaatgt ccacaaagag gagcacgctc atgcccacaa caaggattac 1260
gatatcccaa cgaccgaaaa cctgtatttt cagggatcca gcggctcctc gggaatgacc 1320
gaatacaaac tggttgtagt tggcgctggt ggtgtaggca aaagcgcgct gaccattcag 1380
ttgatccaga accacttcgt agatgagtac gacccgacta ttgaagactc ttaccgtaag 1440
caggttgtta tcgacggtga gacctgtttg ctggacatcc ttgataccgc aggccaagaa 1500
gaatactctg ctatgcgtga tcagtatatg cgtaccggcg aaggcttcct gtgcgttttc 1560
gctatcaaca acaccaaatc ttttgaagac atccatcaat accgtgaaca gatcaaacgt 1620
gttaaagact ctgatgacgt tccgatggtt ctggttggta acaaatgcga cttggcagcg 1680
cgtactgttg aatctcgtca ggctcaggat ctggctcgtt cttacggaat tccgtacatc 1740
gaaacctctg ctaaaactcg tcaaggcgtt gaagacgctt tctacacctt ggttcgtgaa 1800
atccgtcagc acaagctgcg taagctttga tagaattccg tgatagctcg agtcgaccgg 1860

ctgctaacaa agcccgaaag gaagctgagt tggctgctgc caccgctgag caataactag 1920

cataacccct tggggcctct aaacgggtct tgaggggttt tttgctgaaa ggaggaacta 1980

tatccggata acctcgagct gcaggcatgc aagcttggca ctggccgtcg ttttacaacg 2040

tcgtgactgg gaaaaccctg gcgttaccca acttaatcgc cttgcagcac atcccccttt 2100

cgccagctgg cgtaatagcg aagaggcccg caccgatcgc ccttcccaac agttgcgcag 2160

cctgaatggc gaatgcgatt tattcaacaa agccgccgtc ccgtcaagtc agcgtaatgc 2220

tctgccagtg ttacaaccaa ttaaccaatt ctgattagaa aaactcatcg agcatcaaat 2280

gaaactgcaa tttattcata tcaggattat caataccata tttttgaaaa agccgtttct 2340

gtaatgaagg agaaaactca ccgaggcagt tccataggat ggcaagatcc tggtatcggt 2400

ctgcgattcc gactcgtcca acatcaatac aacctattaa tttcccctcg tcaaaaataa 2460

ggttatcaag tgagaaatca ccatgagtga cgactgaatc cggtgagaat ggcaaaagct 2520

tatgcatttc tttccagact tgttcaacag gccagccatt acgctcgtca tcaaaatcac 2580

tcgcatcaac caaaccgtta ttcattcgtg attgcgcctg agcgagacga aatacgcgat 2640

cgctgttaaa aggacaatta caaacaggaa tcgaatgcaa ccggcgcagg aacactgcca 2700

gcgcatcaac aatattttca cctgaatcag gatattcttc taatacctgg aatgctgttt 2760

tccctgggat cgcagtggtg agtaaccatg catcatcagg agtacggata aaatgcttga 2820

tggtcggaag aggcataaat tccgtcagcc agtttagtct gaccatctca tctgtaacat 2880

cattggcaac gctacctttg ccatgtttca gaaacaactc tggcgcatcg ggcttcccat 2940

acaatcgata gattgtcgca cctgattgcc cgacattatc gcgagcccat ttatacccat 3000

ataaatcagc atccatgttg gaatttaatc gcggcttcga gcaagacgtt tcccgttgaa 3060

tatggctcat aacacccctt gtattactgt ttatgtaagc agacagtttt attgttcatg 3120

atgatatatt tttatcttgt gcaatgtaac atcagagatt ttgagacaca acgtggcttt 3180

gttgaataaa tcgaactttt gctgagttga aggatcagat cacgcatctt cccgacaacg 3240

cagaccgttc cgtggcaaag caaaagttca aaatcaccaa ctggtccacc tacaacaaag 3300

ctctcatcaa ccgtggctcc ctcactttct ggctggatga tggggcgatt caggcctggt 3360

atgagtcagc aacaccttct tcacgaggca gacctc 3396